# EUROPEAN PATENT SPECIFICATION

(11) **EP 2 154 824 B1**
(45) Date of publication and mention of the grant of the patent: **08.02.2012**
(21) Application number: 09172242.1
(22) Date of filing: 29.10.2003
(51) Int. Cl.: H04L 12/24, G06F 19/00, G06F 17/50, G06F 17/30

(54) **Method and apparatus for identifying components of a network having high importance for network integrity**
Verfahren und Vorrichtung zum identifizieren von Komponenten eines Netzwerks mit grosser Wichtigkeit für die Netzwerkintegrität
Procédé et appareil pour identifier des éléments d'un réseau ayant une grande importance pour l'intégrité du réseau

(30) Priority: 29.10.2002 GB 0225109
(43) Date of publication of application: 17.02.2010
(62) Divisional of application: 08167881.5
(73) Proprietor: E-Therapeutics plc, Newcastle Upon Tyne NE2 4LZ (GB)
(72) Inventor: Young, Malcolm Philip, Newcastle upon Tyne, Tyne and Wear NE99 (GB); Andras, Peter Emil, Newcastle upon Tyne, Tyne and Wear NE99 (GB); O'Neill, Mark Anthony, Newcastle upon Tyne, Tyne and Wear NE99 (GB)
(74) Representative: Fox, Nicholas Russell Philip

(56) References cited:
- US-A- 6 038 390
- SCHROEDER M A ET AL: "Enhanced network survivability through balanced resource criticality" 19891015; 19891015 - 19891018, 15 October 1989 (1989-10-15), pages 682-687, XP010083531
- NOAKES M D ET AL: "An adaptive link assignment algorithm for dynamically changing topologies" 19881023; 19881023 - 19881026, 23 October 1988 (1988-10-23), pages 683-689, XP010072012
- HELDEN VAN J ET AL: "REPRESENTING AND ANALYSING MOLECULAR AND CELLULAR FUNCTION USING THE COMPUTER" BIOLOGICAL CHEMISTRY, WALTER DE GRUYTER GMBH & CO, BERLIN, DE, vol. 381, no. 9/10, 1 September 2000 (2000-09-01), pages 921-935, XP008032491 ISSN: 1431-6730

## Description

The present invention relates to methods of analysing networks of interconnected components to identify components of a network which are of high importance for maintaining the network's integrity. The invention also relates to apparatus for carrying out such methods.

Many sorts of systems can be represented in the form of networks comprising nodes interconnected by links. Examples of such networks are social interactions where the nodes might be individuals and the links interactions between those individuals, the Internet where nodes are computers and the links are communication links between computers, and proteome data where nodes indicate proteins and links indicate exchanges of metabolites or interactions between the proteins.

It has been found that in complex systems often a relatively small proportion of the components in a complex system are vital to its function. Thus for example most single protein species in an intra cellular metabolic network can be removed without affecting the function of the system, as can individual exchanges in a telecommunications network. The reason for this is that there are frequently many alternative routes around any removed or dysfunctional element in a complex system, which alternative routes can yield the same metabolic, physical or informational result.

It is therefore desirable to provide a computer system which can analyse data representative of a network to identify those components which are of high importance for network integrity. In the case of a communications network, if such components can be identified, additional backup can be built to protect the functioning of the vital nodes. In the case of network data representing the proteome of a living organism, the identification of important elements in a network representing the proteome enables potential targets for drug intervention to be identified.

Schroeder M A et al: "Enhanced network survivability through balanced resource criticality" 15 October 1989 pages 882-687discloses processing network data representing a military hierarchy to identify low redundancy links. To assess the level of redundancy of a link the total number of paths between nodes in calculated and the proportion of those paths which pass through a particular link is determined.

Helden Van J et al : "Representing and Analysing Molecular and Cellular Functions using the computer" Biological Chemistry, Walter de Gruyter GmbH & Co. Berlin, vol. 381, no. 9/10, 1 September 2000 pages 921-935 discusses computer implemented methods of visualising proteome data.

In accordance with one aspect of the present invention there is provided a computer implemented method of identifying target proteins or protein interactions for targeting by drug therapies comprising: obtaining proteome data for an organism to be targeted; storing said proteome data as network data within a computer , said network data comprising data defining a plurality of nodes and a plurality of links between said nodes, wherein said nodes are associated with proteins and said links are indicative of protein interactions; processing the stored network data utilizing the computer to determine for each of the links of the network defined by the stored network data , a value indicative of the ratio of the number of paths between the nodes in the network which pass via the link relative to the total number of paths between said nodes in said network; and utilizing said determined values associated with said links corresponding to protein interactions to identify one or more proteins or protein interactions as target proteins or protein interactions for targeting by drug therapies.

In accordance with another aspect of the present invention there is provided a computer implemented method of identifying target proteins for targeting by drug therapies comprising: obtaining proteome data for an organism to be targeted; storing said proteome data as network data within a computer , said network data comprising data defining a plurality of nodes and a plurality of links between said nodes, wherein said nodes are associated with proteins and said links are indicative of protein interactions; processing the stored network data utilizing the computer to determine for each of the nodes of the network defined by the stored network data , a value, indicative of the number of paths between the nodes in the network which pass via the nodes relative to the total number of paths between said nodes in said network; and utilizing said determined values associated with said nodes corresponding to proteins to identify one or more proteins as target proteins for targeting by drug therapies.

In accordance with another aspect of the present invention there is provided an information processing apparatus for identifying target proteins or protein interactions for targeting by drug therapies comprising: a data store configured to store proteome data for an organism to be targeted as network data defining a plurality of nodes and a plurality of links between said nodes wherein said nodes are associated with proteins and said links are indicative of protein interactions; a processing unit operable to process network data stored in said data store to determine for each of the links of the network defined by the stored network data , a value indicative of the ratio of the number of paths between the nodes in the network which pass via the link relative to the total number of paths between said nodes in said network; and an output unit operable to output data identifying one or more target proteins or protein interactions for targeting by drug therapies on the basis of the values associated with links determined by the processing unit.

In accordance with a further aspect of the present invention there is provided an information processing apparatus for identifying target proteins for targeting by drug therapies comprising: a data store configured to store proteome data for an organism to be targeted as network data defining a plurality of nodes and a plurality of links between said nodes wherein said nodes are associated with proteins and said links are indicative of protein interactions; a processing unit operable to process network data stored in said data store to determine for each of the nodes of the network defined by the stored network data , a value indicative of the ratio of the number of paths between the nodes in the network which pass via said nodes relative to the total number of paths between said nodes in said network; and an output unit operable to output data identifying one or more target proteins for targeting by drug therapies on the basis of the values associated with nodes determined by the processing unit.

Further aspects and embodiments of the present invention will become apparent with reference to the accompanying drawings in which:
Figure 1 is a schematic diagram of the processing of data representing nodes interconnected by links by a computer to identify network components of high importance for the integrity of a network;
Figure 2 is a flow diagram of a method of utilising the computer of Figure 1 as part of a system for identifying pharmaceutical compounds and screening those compounds to identify effective drug treatments;
Figure 3 is a schematic block diagram of the computer modules stored within the memory of the computer of Figure 1;
Figure 4 is a flow diagram of the processing of the computer of Figure 1;
Figure 5 is a schematic block diagram of network data stored within the memory of the computer of Figure 1;
Figure 6 is a schematic illustration of a portion of a network identifying a "hub" node;
Figure 7 is a schematic illustration of a portion of a network identifying a low redundancy node in a network;
Figure 8 is a flow diagram of the processing of the computer of Figure 1 to identify low redundancy nodes;
Figure 9 is a schematic illustration of an exemplary portion of a network for illustrating the processing of Figure 8;
Figure 10 is a schematic illustration of a portion of network identifying a low redundancy link in a network;
Figure 11 is a flow diagram of the processing of the computer of Figure 1 to identify low redundancy links;
Figure 12 is a schematic illustration of a portion of a network connecting two sub networks;
Figure 13 is a schematic illustration of a portion of a network identifying a second order node; and
Figure 14 is a schematic illustration of a report identifying possible cellular targets for pharmaceutical compounds.

### Overview of System

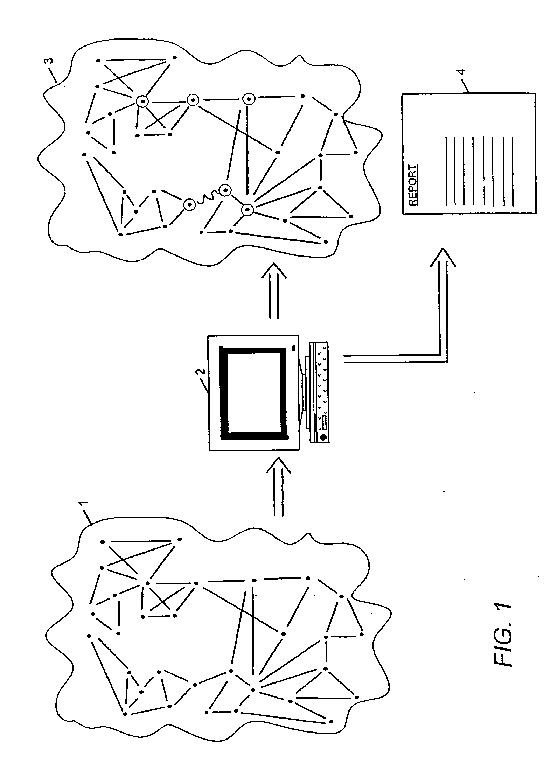

Figure 1 is a schematic illustration of an embodiment of the present invention. In this embodiment data representing a network 1 in the form of nodes (shown as dots in Figure 1) interconnected by links (shown as lines in Figure 1) is input into a computer 2. For illustrative purposes, the data defining a network 1 in this embodiment is taken to be data defining a proteome. That is to say in this embodiment the nodes are indicative of proteins within an organism and the links identify which proteins interact with one another.

Once data representing a network (proteome) has been input into the computer 2, the computer 2 processes the data representing the network to identify within the network a series of nodes and links which are of particular importance for the structural integrity of the network. In Figure 1, processed data is illustrated by network 3 where the identified nodes of importance for structural integrity of the network are illustrated by circles highlighting some of the nodes. In addition, in Figure 1 a link in the network is highlighted as a critical link by a wavy line in the output data 3.

The nodes and links identified as being of importance to the structural integrity of the network 1 by the processing of the computer 2 are established in a number of different ways. Once the critical nodes and links have been identified by the processing of the computer 2, the computer 2 can then output a report 4 identifying the critical nodes and links. In the case of a network 1 representing a proteome of an organism, this report will identify potential drug targets for disrupting the functioning of the organism the proteome 1 represents.

The pharmaceutical industry faces the difficult task of identifying cellular targets for drug intervention. Ordinarily, in a particular cell type, there may be proteome data which identifies between 4000 and 6000 potential proteins which could be possible targets. Checking the effect of disrupting the operation of each protein is therefore very time consuming and expensive, particularly as normally organisms are able to compensate for the disruption of individual proteins.

Previously, heuristic approach and serendipity have been the only means of focussing on potential targets for intervention which are likely to yield biological effects when intervened upon by pharmaceutical chemicals. Targeting multiple proteins in a drug treatment can be more successful. However, the number of potential combinations which could be tested is enormous. Given the costs involved, a more focussed approach is desirable.

The applicants have appreciated that certain topological features of a network enable certain nodes and links to be identified as likely suitable targets since these nodes and links can be identified as being of importance to the structural integrity of a network represented by node and link data. Further, the applicants have determined methods by which groups of target nodes of importance for structural integrity can be identified.

Further, by having the computer 2 store data identifying the critical proteins which are utilised and conserved in a host organism, as will be described the potential targets identified by the computer can be filtered so that the report 4 suggests target proteins of the organism represented by the proteome 1 which are not conserved or utilised by a host organism and hence are more likely not to cause side effects in a host. Additionally, the computer 2 can be arranged to include in the report 4 details of agents which are known to attack the functions of the identified critical proteins.

### Use of System in Treatment Identification

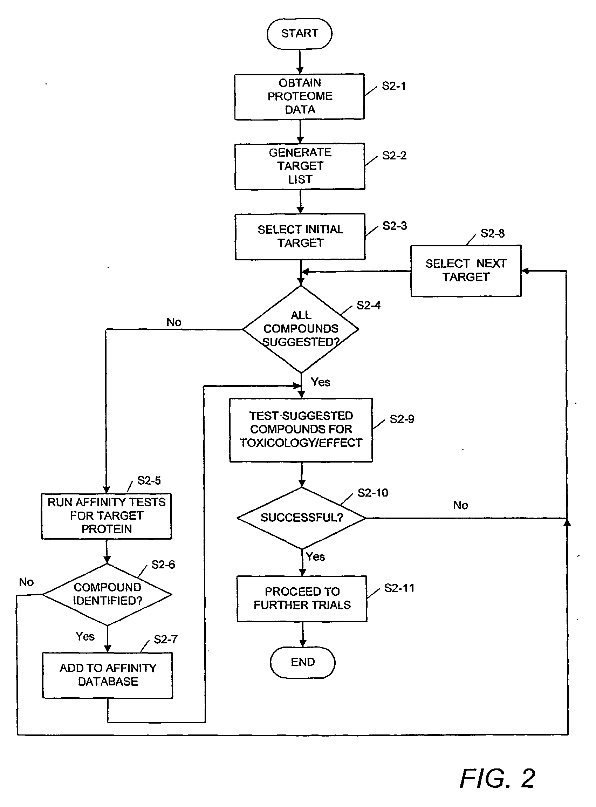

Before describing the structure and functionality of the above computer system 2 in detail, the use of the above system in identifying potential compounds for treating infections will now be described in with reference to Figure 2.

Initially (S2-1) proteome data 1 for a target organism is acquired utilising conventional techniques. This proteome data 1 will identify the proteins present within an organism and also the interactions between those proteins. Identification of the proteins can be achieved using conventional techniques such as mass spectrometry and chromatography etc. Whether different proteins interact can then be established using laboratory techniques such as by manipulating proteins so as to be represented in yeast and seeing whether generated proteins interact. When data for the proteome has been acquired it is then entered into the computer 2 and stored.

The computer 2 then (S2-2) processes the input data in the manner described above so as to generate target data which is output in the form of a report 4. This report will identify lists of potential targets which by virtue of the analysis of the network topology of the stored proteome data 1 will highlight potential targets for intervention.

An initial target identified by the report 4 is then selected (S2-3) and checking the report 4 it is determined (S2-4) whether or not any agents are known to react with the identified protein.

If this is not the case affinity tests can then be run (S2-5) against an expression of the identified protein or proteins to attempt to identify (S2-6) possible compounds that interact with the target. If it is determined that a compound suitable for interacting with the identified target can be found, this data is then added (S2-7) to a compound affinity database for future reference. Alternatively if no such compounds can be found, the next target (S2-8) from the report 4 can be selected for analysis.

Either when one or more compounds for attacking specific targets are suggested by the report 4 or alternatively when suitable compounds have been identified through affinity tests the compound or groups of compounds for targeting the identified protein or proteins can then be tested (S2-9) for toxicology and effect to see whether the combination of compounds does indeed disrupt the activity of the organism represented by the proteome data. If the tests (S2-10) are not successful another set of potential targets from the report 4 can be selected (S2-8) and further potential compounds for therapies can be identified.

If the selected compounds have a desired effect on the organism and are not excessively toxic further trials (S2-11) for the identified compounds can be undertaken to establish whether indeed the identified set of compounds is an effective treatment.

### Structural Components of Computer System

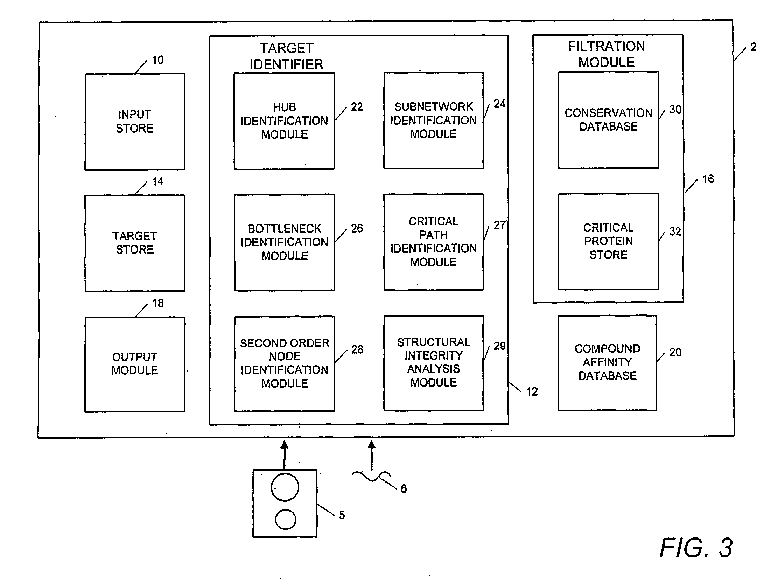

The structure of the computer system of Figure 1 will now be described in detail. Referring to Figure 3 which is a schematic block diagram of the memory of the computer of Figure 1, the computer 2 is programmed to operate in accordance with programming instructions input for example as data stored in a data storage medium such as a disc 5 and/or as a signal 6 input into the computer 2 for example from a remote database by transmission over a communications network (not shown) such as the Internet.

As will be described in more detail below, the programming instructions comprise instructions to cause the memory of the computer 2 to become configured to process input data defining nodes and links in a network. The input data is then processed to generate data identifying critical nodes and links within the network. In the case of input network data defining a proteome where the nodes represent proteins and link interactions between proteins, the identified critical nodes and links will then provide information about potential drug targets.

When programmed by the programming instructions, the memory of the computer 2 effectively becomes configured into a number of functional units for performing processing operations. Examples of such functional units are shown in Figure 3. The units illustrated in Figure 3, are however, notional and are shown for illustration purposes only to assist understanding; they do not necessarily represent exact units and connections into which the processor, memory etc of the computer 2 become configured.

Referring to the functional units shown in Figure 3, an input store 10 is provided for storing data defining network data. In this embodiment which is arranged to process proteome data, this network data comprises data identifying proteins in an organism and known interactions between those proteins.

A target identifier 12 is provided which is arranged to process the network data stored within the input store 10 to identify critical proteins and protein interactions having high importance for the integrity of the proteome. Data identifying the critical proteins is then stored within a target store 14. When the target identifier 12 has stored within the target store 14 data identifying critical proteins, the data within the target store 14 is then filtered utilising a filtration module 16 to identify critical proteins and proteins which are conserved within a host organism.

Finally, an output module 18 utilises the filtered data within the target store 14 and a compound affinity database 20 containing data identifying compounds known to react with proteins to generate and output a report 4 which could be displayed on a screen (not shown) or printed on a printer (not shown) listing the identified critical proteins together with suggested compounds for therapies based on drug targets identified by the target identifier 12.

In this embodiment, the target identifier 12 comprises six sub modules 22-29 each arranged to identify a different type of structure within network data which is indicative of particular components in the network being of high importance for the structural integrity of the network.

The sub modules comprise a hub identification module 22 which is arranged to identify proteins which interact with large numbers of other proteins; a sub network identification module 24 for identifying connections between sub networks; a bottleneck identification module 26 and a critical path identification module 27 for identifying nodes and links within the network data in the input store 10 which cannot be easily bypassed and hence are of importance for the integrity of the network; a second order node identification module 28 for identifying nodes representing proteins directly interacting with nodes identified by the hub identification module 22, sub network identification module 24 and bottleneck identification module 26; and a structural integrity analysis module 29 for identifying groups of nodes which together significantly affect the structural integrity of the network represented by the data within the input store 10.

As will be described after targets and proteins have been identified by the target identifier 12 and stored within the target store 14, the targets are filtered by a filtration module 16. In this embodiment, the filtration module 16 comprises a conservation database 30 and a critical protein store 32.

The conservation database 30 is arranged to store data identifying similar proteins which are conserved between different organisms. Thus for example data is stored identifying that a particular protein in an organism is substantially a homolog of another protein in a host such as a human. The critical protein store 32 is a database storing data identifying critical proteins for the activity of a host. When data identifying a number of target proteins has been generated and stored within the target store 14, the stored targets are likely to be proteins and metabolites which will disrupt the activity of the organism identified by the proteome data and the input store 10 by virtue of the manner of the processing by the target identifier 12. However, although such targets may be useful for enabling general disinfectants to be identified, if a suitable drug is to be developed it must not only be effective against a target organism, but also must not have excessive side effects.

In order to aid with the identification of more promising drug targets, the filtration module 16 stores in the conservation database 30 data for identifying which proteins have similar proteins in the host organism. Where a potential target protein is identified which is not present in any form in a host organism it is more likely that a therapy disrupting that particular protein will have limited side effects. If it is not possible to identify a protein which is not conserved as a potential target, at the very least it is desirable to ensure that the targets chosen for further research are unlikely to disrupt the critical systems of a host. By storing data in a critical protein store 32 identifying the critical proteins for a host the list of potential targets can be appropriately filtered to highlight the most promising potential therapies.

### Processing to Identify Critical Nodes and Links

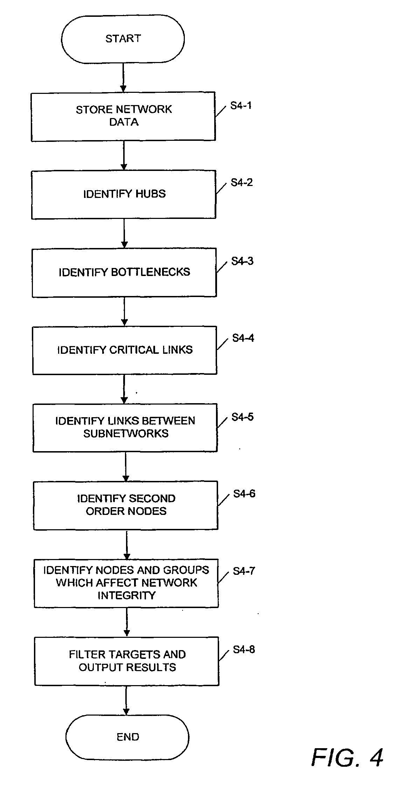

The processing of the computer 2 will now be described in greater detail with reference to Figure 4 which is a flow diagram illustrating the processing of the computer 2.

Initially (S4-1) data representing the network to be analysed is stored within the input store 10.

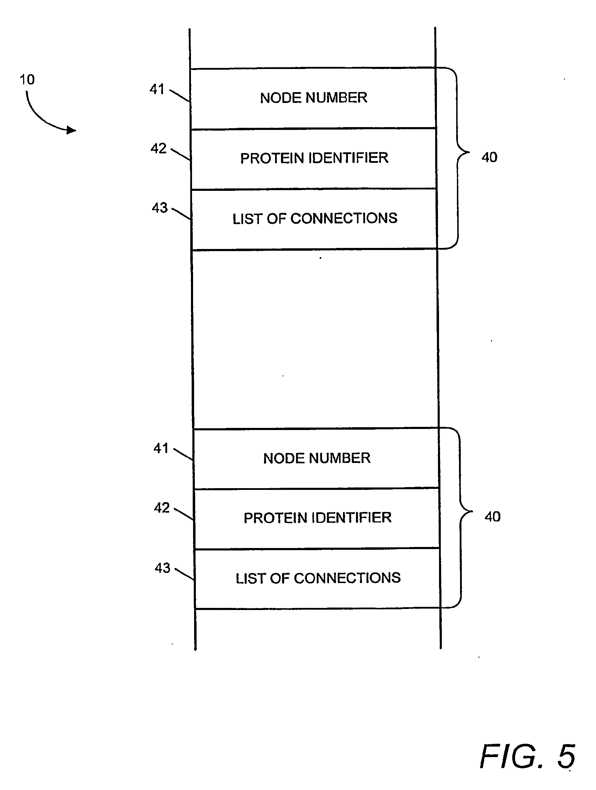

Figure 5 is a schematic illustration of data stored within the input store 10. In this embodiment the data stored within the input store 10 is stored in the form of a number of node records 40 each comprising a node number 41, a protein identifier 42 and a list of connections 43. One of these records 40 is stored for each of the proteins within the proteome being analysed. In each record 40 the list of connections 43 is a list of node numbers 41 of the node records 40 of the proteins with which the protein identified by the protein identifier 42 for the record 40 is known to interact with. Such data can be obtained for a proteome for a particular organism or cell utilising conventional laboratory techniques.

In other embodiments where the network data stored within the input store 10 is representative of a network other than a proteome, the protein identifier 42 will be replaced with a different identifier of a network component and the list of connections 42 would be a list of node numbers 41 of components within the network an identified component interacts with.

### (a) Hub Identification

Once data for the proteome has been stored within the input store 10, the target identifier 12 then invokes the hub identification module 22 to identify (S4-2) hub nodes within the network.

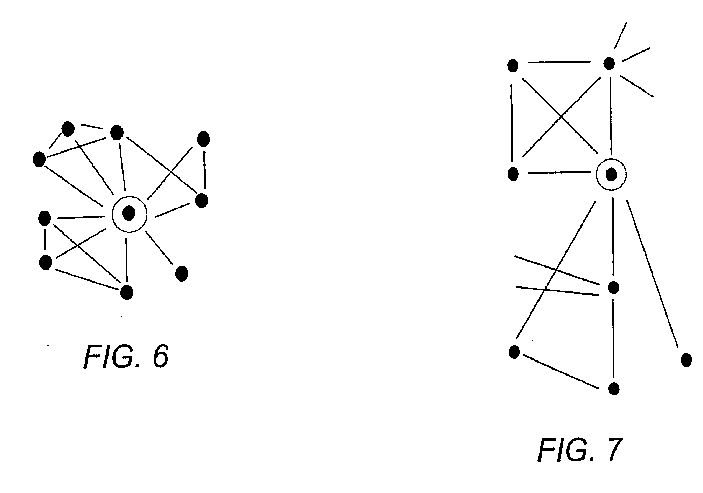

Figure 6 is a schematic illustration of a portion of a network. In Figure 6 nodes are represented by circles and links between nodes are indicated by lines connecting the circles. As shown in Figure 6 some nodes such as the node highlighted by a larger circle interact with significantly more nodes than average. Where nodes represent proteins such well connected nodes are often indicative of proteins critical to the functioning of an organism. By identifying such nodes, potential drug targets can therefore be found.

In this embodiment which is arranged to process proteome data where normally approximately around about 4000-6000 proteins are included in a proteome and hence 4000-6000 node records 40 will ordinarily be stored in the input store 10. When this number of nodes is stored the hub identification module 22 in this embodiment is arranged to store within the target store 14 the node numbers identifying the twenty nodes having the greatest number of entries in their list of connections 43.

### (b) Bottleneck Identification

Returning to Figure 4, after storing data indicative of the hub nodes in the target store 14, the target identifier 12 then (S4-3) invokes the bottleneck identification module 26 to identify within the network represented by data stored within the input store 10 further portions of a network which are important for the structural integrity of that network.

Specifically, the bottleneck identification module 26 is arranged to identify nodes in the network which cannot be easily bypassed. An example of such a node within a network is illustrated in the exemplary network of Figure 7 where all the paths from the nodes shown as dots in the network of Figure 7 pass through a single node highlighted by a circle. If communication through the node highlighted by the circle is disrupted this then has a significant impact on the integrity of the rest of the network as many nodes will no longer be able to communicate with one another.

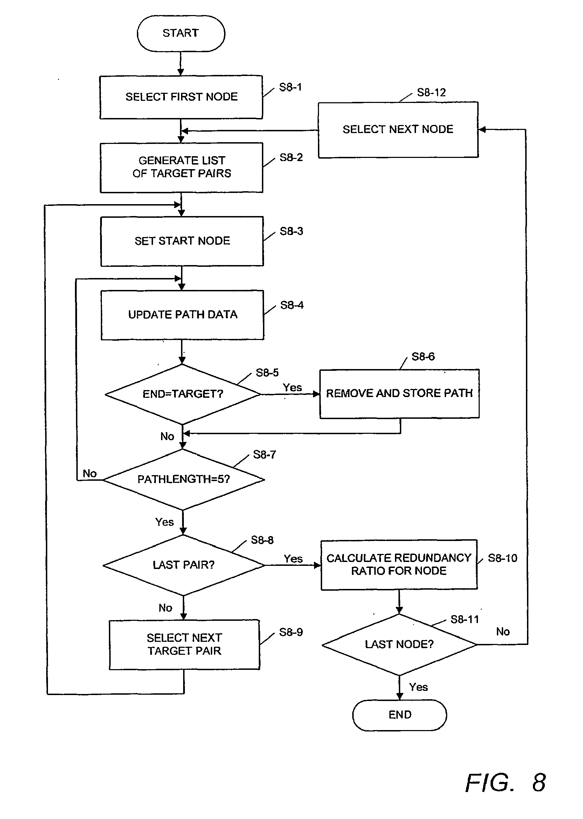

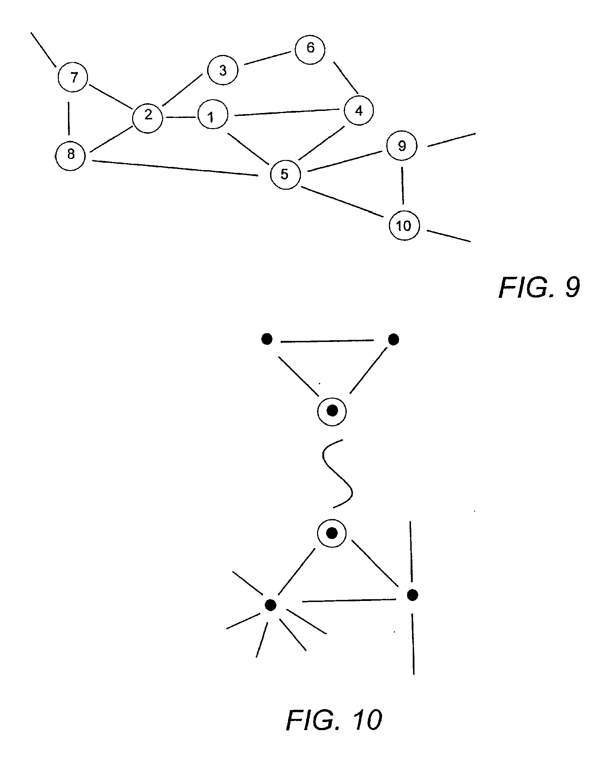

The processing of the bottleneck identification module 26 will now be described in greater detail with reference to Figures 8 and 9 which are a flow diagram of the processing of data by the bottleneck identification module 26 and a schematic illustration of a portion of an exemplary network respectively.

Referring to Figure 8, when the bottleneck identification module 26 is initially invoked (S8-1) the bottleneck identification module 26 selects a first node for processing. In this embodiment this is achieved by the bottleneck identification module 26 selecting the node record 40 having a node number 41 equal to one.

Thus for example, in the illustrative network of Figure 9 where nodes are indicated by numbers surrounded by circles and links between nodes are shown as lines between the circles, the bottleneck identification module 26 would select for processing the node identified by the number 1 in Figure 9. The bottleneck identification module 25 then (S8-2) generates a list of target pairs. Specifically, the bottleneck identification module 26 processes the list of connections 43 of the node record 40 currently being processed and generates a set of target pairs comprising pairs of distinct node numbers identified from the list of connections 43.

Thus for example, in the case of the exemplary network of Figure 9 where node 1 is shown as being connected to nodes 2, 4 and 5, the node record 40 having a node number 41 set equal to 1 would have a list of connections 43 of the following form [2,4,5]. The bottleneck identification module 26 would therefore generate as a list of target pairs the following set of target pairs [(2,4),(2,5), (4,5) ].

Once a list of target pairs has been generated, the bottleneck identification module 26 then (S8-3) selects the first target pair and sets as a start node the first value in the target pair. The bottleneck identification module 26 then generates an initial item of path data comprising a list consisting of this selected start node.

Thus in the case of processing the target pair (2,4) the bottleneck identification module 26 would select as a start node the node number 2 and generate a single item of path data comprising list: [2 ].

The bottleneck identification module 26 then proceeds to process all the currently existing items of path data by taking each of the items of path data in turn. For each item of path data, the final entry in the list of nodes comprising the path data is then identified. The item of path data is then replaced by a number of items of path data consisting of the current item of path data to which is appended data representative of the different nodes from the list of connections 43 for the node record 40 of the last entry in the item of path data being processed.

Thus, in the case of processing the item of path data consisting of a single entry [2] and the exemplary network of Figure 9, the list of connections 43 for the node record 40 having a node number set equal to 2, would be [1,3,7,8]. When processing the item of path data [2 ], this item of path data would therefore be replaced by the following items of path data:
[2,1]
[2,3]
[2,7]
[2,8]

The bottleneck identification module 26 then checks each of the newly generated items of path data and deletes any items of path data which contain any node number more than once. In the case of the above exemplary list of generated items of path data, since none of these contain a node number more than once no items of path data would be deleted.

When all of the existing path data has been updated, the bottleneck identification module 26 then (S8-5) determines whether the final entry in any of the newly generated items of path data corresponds to the second value of the target pair currently being processed.

Thus in the case of processing the target pair (2,4) the bottleneck identification module 26 would check whether any of the entries in each of the generated items of path data was equal to 4.

If this is found to be the case, the bottleneck identification module 26 then removes the identified item of path data from further processing and stores it separately for later consideration (S8-6).

After any items of path data having a final value equal to the second entry in the target pair being processed has been identified and stored, the bottleneck identification module 26 checks (S8-7) whether path data having five entries has been generated. If this is not the case, the bottleneck identification module 26 then processes the currently existing items of path data in the same way as has previously been described (S8-4-S8-6), generating new items of path data by appending further node numbers to the existing items of path data before checking once again whether the current length of items of generated path data is now equal to five entries (S8-7).

Thus in the case of processing the items of path data described above at the second iteration when processing the target pair (2,4), the following items of path data would be generated: [2,1,4 ] ,[2,1,5 ,[2,3,6 ],[2,7,8 ], [2,8,5 ],[2,8,7 ] of which the path data [2,1,4 ] would be identified as ending with the value 4 and stored separately for later processing.

Eventually, the bottleneck identification module 26 will determine that path data having five entries has been generated. At this stage, the bottleneck identification module 26 will have stored path data identifying every path between nodes identified by the current target pair having no more than five elements.

In the case of processing the target pair (2,4) of the exemplary network of Figure 9, the following data would therefore have been stored: [2,1,4 ],[2,8,5,4 ],[2,7,8,5,4 ],[2,3,6,4 ],[2,8,5,1,4 ].

The bottleneck identification module 26 then (S8-8) checks whether the target pair being processed is the final target pair generated for the current node. If this is not the case, the next target pair is then selected (S8-9) and processed in the same way as the previous target pair (S8-3-S8-8). As a result further path data, identifying paths between the two nodes identified by the next target pair will be generated and stored.

Thus in the case of the example network of Figure 9, processing the target pair (2,5) would cause the following items of path data to be stored: [2,1,5] ,[2,3,6,4,5 ],[2,7,8,5 ],[2,8,5 ],[2,1,4,5 ].

When the bottleneck identification module 26 determines (S8-8) that all generated target pairs for a particular node have been processed, the bottleneck identification module 26 then (S8-10) proceeds to use the stored items of path data to calculate a redundancy ratio for the node being processed.

Specifically, the bottleneck identification module 26 determines the number of stored items of path data which include the current node being processed relative to the total number of stored items of path data.

Thus in the case of processing node 1 of Figure 9 where the following path data would be stored: [2,1,4 ],[2,8,5,4 ],[2,7,8,5,4 ],[2,3,6,4 ],[2,8,5,1,4 ],[2,1,5 ],[2,3,6,4,5 ],[2,7,8,5 ],[2,1,8,5 ],[4,1,5 ],[4,5 ],[4, 1,2,8,5 ] a redundancy ratio of 6/12 = 0.5 would be determined.

This value is indicative of the proportion of paths between nodes connected to the node for which the ratio is calculated which pass through that node. Thus in the case of a high ratio value, this will indicate that there are very few paths which can bypass that node and hence that the node is of relatively high importance for the structural integrity of that portion of the network.

The value therefore provides an indication of whether disruption of the node is likely to disrupt communication through the network.

After the bottleneck identification module 26 has calculated a redundancy ratio for a node, the bottleneck identification module 26 checks (S8-11) whether a redundancy ratio has been calculated for all the nodes in the network. If this is not the case, the next node record 40 for the next node number 41 is selected for processing (S8-12) and a redundancy ratio for that node is determined (S8-2-S8-10) before the bottleneck identification module 26 checks once again (S8-11) that redundancy ratios for all nodes have been calculated.

When a redundancy ratio for each of the nodes has been determined, the bottleneck identification module 26 can then use the stored redundancy ratio data to identify the nodes in the network which cannot easily be bypassed. In this embodiment data identifying the node numbers of the nodes associated with the redundancy ratios indicating the twenty nodes which are hardest to bypass is then stored in the target store 14.

### (c) Critical Link Identification

Returning to Figure 4, after the bottleneck identification module 26 has identified and stored data identifying any nodes which are difficult to bypass in the network, the critical path identification module 27 is then invoked and attempts to identify (S4-4) individual links within the network which are difficult to bypass.

Figure 10 is a schematic illustration of a portion of a network where a critical link between two nodes is highlighted. In the case of Figure 10 the highlighted nodes are surrounded by larger circles and the highlighted link is illustrated by a wavy line.

When processing data to identify nodes that are difficult to bypass such as that illustrated in Figure 10, often these critical nodes will be connected to one another. In such circumstance in addition to identifying the nodes as of importance for the structural integrity of the network, the individual link between two nodes can also be identified as a potential weakness within the network.

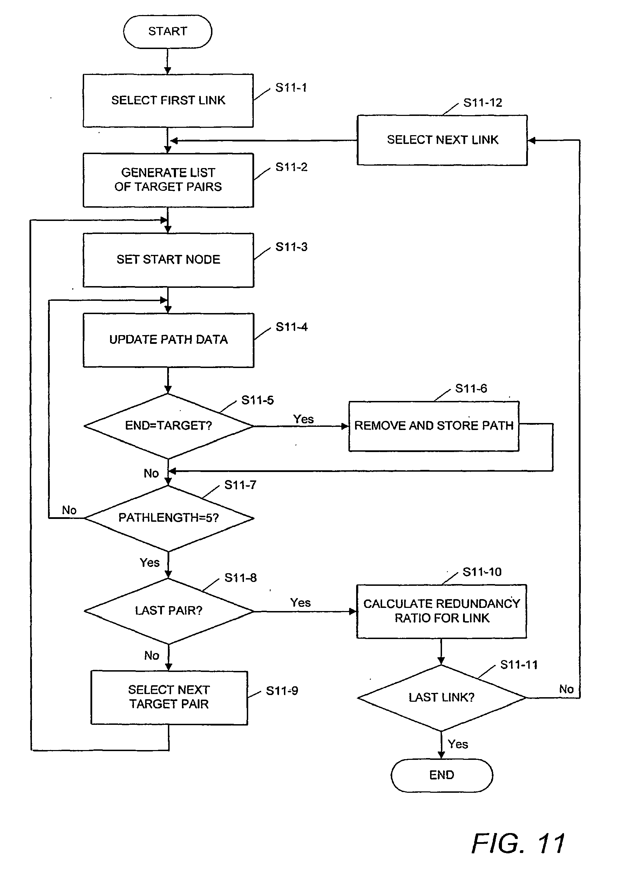

The processing of the critical path identification module 27 will now be described in detail with reference to Figure 11 which is a flow diagram of the processing of the critical path identification module 27.

The processing of the critical path identification module 27 is very similar to the processing undertaken by the bottleneck identification module 26. However, instead of processing each of the nodes in turn, the critical path identification module 27 processes each link within the network.

When the critical path identification module is first invoked a first link (S11-1) is selected. In this embodiment this link is the link identified by the node number 41 of the first node record 40 and the first entry in the list of connections 43 associated with that node record 40 where the identified entry in the list of connections 43 is a node number no greater than the node number 41 for the node record 40 currently being processed.

Thus for example processing the network of Figure 9, the node record 40 for node 1 would be selected and then the link [1-2] would be identified for processing.

The critical path identification module 27 then generates a list of target pairs (S11-2) in a similar way to the generation of target pairs previously described in relation to the processing of the bottleneck identification module 26. However, in this case instead of generating a list of target pairs utilising the nodes identified in the list of connections 43 of the node record 40 currently being processed, the critical path identification module 27 generates a set of target pairs utilising the lists of connections 43 of both of the nodes identified by the link currently being processed. This list of target of pairs is generated by determining all possible distinct pairs of nodes that can be formed by selecting entries from the lists of connections 43 of the two node records 40 identified by the link.

Thus in the case of the exemplary network of Figure 9 processing the link [1-2 ], the critical path identification module 27 would utilise the list of connections 43 for the first and second nodes namely the lists: [2,4,5] and [1,3,7,8] to generate the following list of target pairs where each of the entries in each pair are distinct:
(2,1) (4,1) (5,1)
(2,3) (4,3) (5,3)
(2,7) (4,7) (5,7)
(2,8) (4,8) (5,8)

After this list of target pairs has been generated for the link being processed, these target pairs are utilised in exactly the same way as has previously been described in relation to the processing of the bottleneck identification module 26. That is to say the target pairs are used to generate and store a series of items of path data including up to five entries where the head and tail of each list corresponds to a head and tail of one of the target pairs (S11-3-S11-9). In this way the critical path identification module 27 identifies every path of up to four links between each of the nodes connected to the nodes of the link currently being processed.

When path data has been generated and stored for all of the target pairs generated for a particular link, the critical path identification module 27 then (S11-10) calculates a redundancy ratio for the link. This is achieved in a similar way to the calculation of a redundancy ratio by the bottleneck identification module 26. However in the case of the critical path identification module 27, the critical path identification module 27, calculates the proportion of stored items of path data which include a step corresponding to the link currently being processed.

Thus for example when generating a redundancy ratio value for the link (1,2) the critical path identification module 27 determines the proportion of stored of items of path data for a link including within the path data either the entry 2 followed by the entry 1 or the entry 1 followed by the entry 2.

Once a redundancy ratio for a particular link has been calculated, the critical path identification module 27 then checks (S11-11) whether all of the links in the network have been processed. If this is not the case, the critical path identification module 27 then (S11-12) selects the next link for processing and calculates (S11-2-S11-11) a redundancy ratio for that link.

When all of the links have been processed, a redundancy value will be stored for each of the links where a high redundancy ratio values indicates a link within the network which cannot easily be bypassed. Data identifying the 20 links associated with the highest redundancy ratio values is then stored within the target store 14 together with data identifying the nodes identified by those links.

### (d) Identification of Links Between Sub Networks

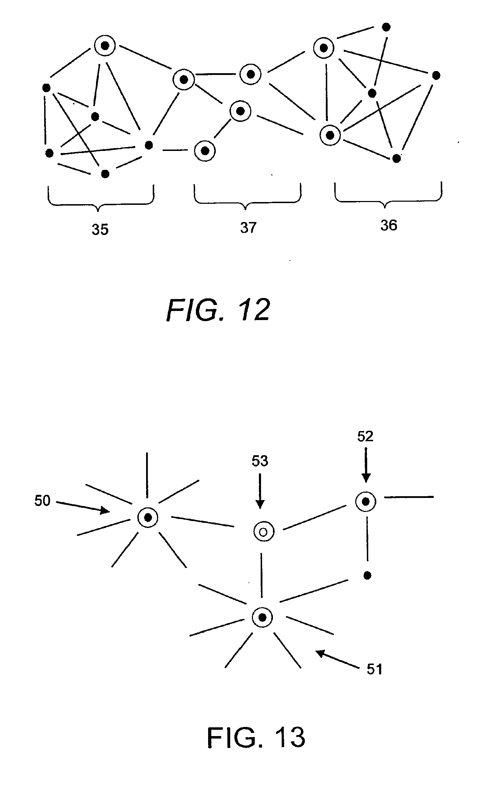

Returning to Figure 4, after the critical path identification module 27 has identified links within the network which cannot easily be bypassed, the sub network identification module 24 is then invoked which then proceeds to identify (S4-5) nodes and links involved in connecting sub networks as will now be described in detail with reference to Figures 12-15.

Figure 12 is a schematic illustration of a network divided into two sub networks. In this application the term sub network is taken to mean portions of a network comprising nodes that are more connected to one another than other nodes in the rest of the network. Thus in the case of Figure 12 the left and right hand sections of the illustrated network 35,36 are examples of sub networks whereas the nodes in the centre of the illustrations 37 are an illustrative example of a bridge between two sub networks. That is to say the nodes shown as highlighted provide a connection between the two sub networks 35,36.

When network data is representative of for example a proteome, the existence of sub networks normally identify a series of proteins and protein interactions responsible for different functions within the organism. Thus for example one sub network might involve proteins responsible for controlling cell division, whereas another sub network might identify proteins responsible for controlling energy generation.

By identifying nodes responsible for linking the activities of two sub networks, it is possible to identify targets which disturb communications between the sub networks. In the case of an organism, this could for example cause the functions responsible for cell division to no longer be co-ordinated with the energy generation network and hence cause the organism to no longer reproduce properly.

### (e) Identification of Second Order Nodes

At this stage stored within the target store 14 is data identifying hub nodes, nodes that are difficult to route around and nodes involved in links between sub networks. Each of the sets of nodes will have been identified utilising the node and link data defining a network topology stored in the input store 10.

In addition to these nodes, the applicants have appreciated that a further set of nodes that are important for network integrity are those nodes that are connected to these identified hubs, nodes that are difficult to avoid and links between sub networks. This is because these nodes interact with nodes of importance and hence if the functioning of these connected nodes is disrupted, the functioning of the other identified nodes of importance may also be effected.

In the case of proteome data identifying proteins and protein interactions, frequently, certain proteins corresponding to hubs or other critical nodes are in practice unsuitable for targets as disrupting the activity of such protein can cause unwanted side effects in a host. The secondary proteins which interact with these critical proteins may, however, differ between a target organism and a host. By interfering with the manner in which these proteins interact with the identified critical nodes, the activities of these critical nodes can be effected in a way does not cause a corresponding disruption of the activity of a host. Thus for example in Figure 13 there are four nodes, two of these nodes labelled 50 and 51 are examples of hub nodes having many connections. Node 52 is shown as an example of a node which is difficult to route around. In the exemplary network of Figure 16, node 53 is shown as being connected to nodes 50, 51 and 52, all of which can be identified as being of potential importance by virtue of analysis of the network topology. Given the large number of links node 53 has to nodes identifiable as important, enables node 53 to be identified as a potential target for effecting the structural integrity of the network.

Thus returning to Figure 4, in this embodiment once the hub nodes, nodes linking sub networks and nodes which are difficult to route around have been identified, the target identifier 12 invokes the second order node identification module 28. The second order node identification module 28 then (S4-6) determines for each of the nodes in the network the number of nodes for which identifying data has been stored in the target store 14 which are contained in the list of connections 43 in each of the node records 40. This data is stored for each of the nodes and the second order node identification module 28 then identifies the top twenty nodes connected to the greatest number of other nodes of importance. Thus in this way the second order node identification module 28 is able to identify those nodes which are directly linked to a number of other nodes of importance.

### (f) Identification of Groups of Nodes for Affecting Network Integrity

At this stage, the target store 14 will have stored within it data identifying the node numbers of all of the nodes identified by the hub identification module 22, sub network identification module 24, bottleneck identification 26, critical path identification module 27 and second order node identification module 28. Although this data identifies individual nodes of importance for maintaining the structural integrity of the network identified by data stored within the input store 10, it is desirable for the target identifier 14 to additionally generate data identifying groups of nodes which together effect the structural integrity of the network. In this embodiment this is achieved by the structural integrity analysis module 29 which proceeds to identify (S4-7) nodes and groups of nodes which effect network integrity.

At this stage as a result of the processing of the target identifier 12, the target store 14 will have stored data identifying hub nodes, nodes involved in connections between sub networks, nodes which are difficult to route around, links that are difficult to route around or are involved in connections between sub networks and groups of nodes which together significantly effect the structural integrity of the network defined by the network data stored within the input store 10.

### (g) Filter targets and output results

After the processing of the structural integrity analysis module 29 has been completed, the output module 18 is invoked (S4-8) which processes the data stored within the target store 14 utilising the filtration module 16 and the compound affinity database 20 to generate a report 4 as will now be described.

Specifically in this embodiment each of the nodes identified by data within the target store 14 is checked against the conservation database 30 and the critical protein store 32 to determine whether the node number identified by data stored within the target store corresponds to the node number 41 of a node record 40 identifying a protein 42 corresponding to a protein stored within the conservation database 30 or the critical protein store 32.

In this way the output module 28 is able to classify each of the items of data stored within the target store 14 as either relating to critical proteins identified by data within the critical protein store 32, proteins corresponding to proteins identified by the conservation database 30 or neither of these.

The output module 18 then generates and outputs a report 4 which identifies the proteins corresponding to the node numbers stored within the target store 14 where the proteins which are determined not to appear in either of the conservation database 30 or the critical protein store 32 are listed separately from those which are determined to appear in the conservation database 30 or the critical protein store 32.

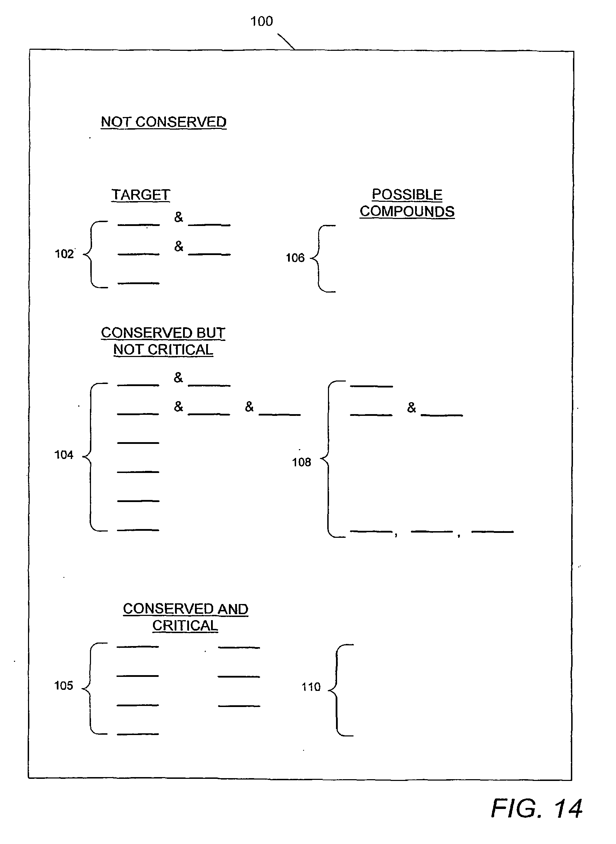

Figure 14 is a schematic illustration of a report 100 generated by the output module 18.

In this embodiment the report 100 comprises three lists 102, 104, 105 where the first list 102 identifies proteins identified by data stored in the target store 14 for which no corresponding entries are stored within the conservation database 30 or critical protein store 32; a second list 104 which identifies proteins identified by data stored within the target store 14 where any of the nodes or nodes within the groups of nodes are identified by data within the conservation database 30 but not the critical protein store 32; and a third list 105 which identifies the remaining proteins identified by data in the target store 14.

In this embodiment adjacent to each of these lists is a further list 106,108,110. Each of these lists identify for the corresponding list within the report 100 any compounds known to react with proteins identified in the list as identified by data within the compound affinity database 20. Thus in this way the output module 18 is able to generate a report where possible target proteins are identified based on an analysis of the topology of network data input into the input store 10.

### Modifications and Amendments

In the above described embodiment, a filtration module 16 is described as including a critical protein store 32 identifying critical proteins for the functioning of a host organism. The data entered into the critical protein store could be obtained through conventional sources. Alternatively the system described could be utilised to identify critical proteins.

Specifically instead of entering proteome data into the input store 10 representative of the proteome of an organism to be attacked, proteome data for the host organism could be entered into the input store. When this host organism proteome data was processed, the target identifier 12 would then proceed to identify hubs, nodes and links involved in connection between sub networks, nodes and links that are difficult to route around and second order nodes and groups of nodes which effect the structure integrity of the network represented in the host organism proteome.

Just as in the case of processing proteome data representative of an organism to be attacked, this processing will identify nodes, links and groups of nodes which are important for the structural integrity of the host organism proteome. By generating data in this way identification of critical proteins, links and groups of protein for a hosting organism could then be achieved.

More generally whenever two networks interact with one another by processing data representative of a first network and storing data identifying critical elements in that first network and then processing data for the second network, it is possible to identify critical elements in the second network, interference with which is less likely to effect the functioning of the first network.

Although in the above described embodiment, the input of data corresponding to a proteome has been described, it will be appreciated that where compounds effecting the interactions of specific proteins have already been identified, proteome data excluding the interactions of a specific protein or group of proteins could be input into the input store 10. The computer 2 would then be able to identify additional targets to complement the activity of the known compound or compounds. Thus in this way when a potential compound has been found to have some activity, complementary targets for therapy could then be identified.

In the above described embodiment, in determining whether a node can be bypassed, determination of a number of paths between connected nodes having five elements is described. It will be appreciated that paths of up to any suitable threshold could be identified and a redundancy ratio calculated on the basis of paths of that length.

Although the embodiments of the invention described with reference to the drawings comprise computer apparatus and processes performed in computer apparatus, the invention also extends to computer programs, particularly computer programs on or in a carrier, adapted for putting the invention into practice. The program may be in the form of source or object code or in any other form suitable for use in the implementation of the processes according to the invention. The carrier be any entity or device capable of carrying the program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal which may be conveyed via electrical or optical cable or by radio or other means.

When a program is embodied in a signal which may be conveyed directly by a cable or other device or means, the carrier may be constituted by such cable or other device or means.

Alternatively, the carrier may be an integrated circuit in which the program is embedded, the integrated circuit being adapted for performing, or for use in the performance of, the relevant processes.

## Claims

1. A computer implemented method of identifying target proteins or protein interactions for targeting by drug therapies comprising:
obtaining (s2-1) proteome data for an organism to be targeted;
storing (s4-1) said proteome data as network data (40) within a computer (2), said network data (40) comprising data defining a plurality of nodes and a plurality of links between said nodes, wherein said nodes are associated with proteins and said links are indicative of protein interactions;
processing (s4-3) the stored network data (40) utilizing the computer (2) to determine for each of the links of the network defined by the stored network data (40), a value indicative of the ratio of the number of paths between the nodes in the network which pass via the link relative to the total number of paths between said nodes in said network; and
utilizing said determined values associated with said links corresponding to protein interactions to identify (s2-2) one or more proteins or protein interactions as target proteins or protein interactions for targeting by drug therapies.

2. A method in accordance with claim 1, wherein said processing (s4-3) comprises, for each of said links:
identifying a first and a second node connected by said link;
identifying a first set of nodes directly connected to said first node and a second set of nodes directly connected to said second node;
identifying (s11-2) all pairs of nodes comprising different nodes selected from said first and said second sets of nodes;
determining (s11-4 - s11-9) for each of said identified pairs of nodes, paths via nodes and links connecting said identified pairs of nodes; and
calculating (s11-10) as said value for a link, the ratio of the number of said determined paths which pass via said link relative to the total number of said determined paths.

3. A method in accordance with claim 2, wherein said determination of paths between pairs of nodes comprises the determination of paths between pairs of nodes having up to a predetermined number of links.

4. A method in accordance with claim 1, wherein utilizing said determined values to identify one or more proteins or protein interactions as target proteins or protein interactions for targeting by drug therapies comprises identifying protein interactions corresponding to links associated with determined values greater than a threshold value.

5. A method in accordance with claim 1, wherein utilizing said determined values to identify one or more proteins or protein interactions as target proteins or protein interactions for targeting by drug therapies comprises identifying proteins corresponding to nodes connected by links associated with determined values greater than a threshold.

6. A computer implemented method of identifying target proteins for targeting by drug therapies comprising:
obtaining (s2-1) proteome data for an organism to be targeted;
storing (s4-1) said proteome data as network data (40) within a computer (2), said network data (40) comprising data defining a plurality of nodes and a plurality of links between said nodes, wherein said nodes are associated with proteins and said links are indicative of protein interactions;
processing (s4-3) the stored network data (40) utilizing the computer (2) to determine for each of the nodes of the network defined by the stored network data (40), a value, indicative of the number of paths between the nodes in the network which pass via the nodes relative to the total number of paths between said nodes in said network; and
utilizing said determined values associated with said nodes corresponding to proteins to identify (s2-2) one or more proteins as target proteins for targeting by drug therapies.

7. A method in accordance with claim 6, wherein said processing (s4-3) comprises, for each of said nodes:
identifying a set of nodes directly connected to a said node via a link;
identifying (s8-2) pairs of nodes of said identified set of nodes;
determining (s8-4 - s8-7) for each of said identified pairs of nodes, paths via nodes and links connecting said identified pairs of nodes; and
calculating (s8-10) as said value for a node, the ratio of the number said determined paths which pass via said node relative to the total number of said determined paths.

8. A method in accordance with claim 7, wherein said determination of paths between pairs of nodes comprises the determination of paths between pairs of nodes having up to a predetermined number of links.

9. A method in accordance with claim 8, wherein said determination of paths between pairs of nodes comprises the determination of paths via nodes wherein said paths do not include any node more than once.

10. A method of manufacturing a therapeutic drug comprising:
identifying one or more target proteins or target protein interactions as target proteins or protein interactions for targeting by drug therapies by performing a method (s2-2) in accordance with any preceding claim;
identifying (s2-6) one or more compounds which react with the indentified one or more target proteins or protein interactions; and
manufacturing a therapeutic drug containing compounds identified as reacting with the identified target proteins or protein interactions.

11. A computer readable medium storing computer interpretable instructions which when interpreted by a programmable computer cause the computer to perform a method in accordance with any of claims 1-9.

12. An information processing apparatus (2) for identifying target proteins or protein interactions for targeting by drug therapies comprising:
a data store (10) configured to store proteome data for an organism to be targeted as network data (40) defining a plurality of nodes and a plurality of links between said nodes wherein said nodes are associated with proteins and said links are indicative of protein interactions;
a processing unit (26) operable to process network data (40) stored in said data store (10) to determine for each of the links of the network defined by the stored network data (40), a value indicative of the ratio of the number of paths between the nodes in the network which pass via the link relative to the total number of paths between said nodes in said network; and
an output unit operable to output data identifying one or more target proteins or protein interactions for targeting by drug therapies on the basis of the values associated with links determined by the processing unit (26).

13. An information processing apparatus (2) in accordance with claim 12 wherein said output unit is operable to output data identifying one or more target proteins for targeting by drug therapies by identifying nodes connected by links associated with determined values greater than a threshold.

14. An information processing apparatus (2) in accordance with claim 12 wherein said output unit is operable to output data identifying one or more target protein interactions for targeting by drug therapies by identifying links associated with determined values greater than a threshold.

15. An information processing apparatus (2) for identifying target proteins for targeting by drug therapies comprising:
a data store (10) configured to store proteome data for an organism to be targeted as network data (40) defining a plurality of nodes and a plurality of links between said nodes wherein said nodes are associated with proteins and said links are indicative of protein interactions;
a processing unit (26) operable to process network data (40) stored in said data store (10) to determine for each of the nodes of the network defined by the stored network data (40), a value indicative of the ratio of the number of paths between the nodes in the network which pass via said nodes relative to the total number of paths between said nodes in said network,; and
an output unit operable to output data identifying one or more target proteins for targeting by drug therapies on the basis of the values associated with nodes determined by the processing unit (26).

## Patentansprüche

1. Ein computerimplementiertes Verfahren zum Identifizieren von Zielproteinen oder Proteininteraktionen zum Targeting von Wirkstofftherapien, umfassend:
Erfassen (S2-1) von Proteomdaten für einen Zielorganismus;
Speichern (S4-1) dieser Proteomdaten als Netzwerkdaten (40) innerhalb eines Computers (2), wobei die Netzwerkdaten (40) Daten umfassen, welche eine Vielzahl von Knoten und eine Vielzahl von Verbindungen zwischen den Knoten definieren, wobei die Knoten mit Proteinen verknüpft sind und die Verbindungen bezeichnend für Proteininteraktionen sind;
Verarbeiten (S4-3) der gespeicherten Netzwerkdaten (40), wobei der Computer (2) genutzt wird, um für jede der Verbindungen des Netzwerks, welches durch die gespeicherten Netzwerkdaten (40) definiert ist, einen Wert zu bestimmen, welcher bezeichnend für das Verhältnis der Anzahl von Wegen zwischen den Knoten in dem Netzwerk, welche über die Verbindung verlaufen, relativ zu der Gesamtzahl von Wegen zwischen den Knoten in dem Netzwerk ist; und
Nutzen der bestimmten Werte, die mit den Verbindungen, welche den Proteininteraktionen entsprechen, verknüpft sind, um ein oder mehrere Proteine oder Proteininteraktionen als Zielproteine oder Proteininteraktionen zum Targeting von Wirkstofftherapien zu identifizieren (S2-2).

2. Verfahren nach Anspruch 1, wobei das Verarbeiten (S4-3) für jede der Verbindungen umfasst:
Identifizieren eines ersten und eines zweiten Knotens, welche durch die Verbindung verbunden sind;
Identifizieren eines ersten Knotensatzes, welcher direkt mit dem ersten Knoten verbunden ist, und eines zweiten Knotensatzes, welcher direkt mit dem zweiten Knoten verbunden ist;
Identifizieren (S11-2) von allen Knotenpaaren, welche unterschiedliche Knoten umfassen, welche von dem ersten und dem zweiten Knotensatz ausgewählt sind;
Bestimmen (S11-4 - S11-9) von Wegen über Knoten und Verbindungen, welche die identifizierten Knotenpaare anschließen, für jedes der identifizierten Knotenpaare; und
Berechnen (S11-10) des Verhältnisses der Anzahl der bestimmten Wege, welche über die Verbindung verlaufen, relativ zu der Gesamtzahl der bestimmten Wege als den Wert für eine Verbindung.

3. Verfahren nach Anspruch 2, wobei die Bestimmung von Wegen zwischen Knotenpaaren die Bestimmung von Wegen zwischen Knotenpaaren, welche bis zu einer vorbestimmten Anzahl von Verbindungen aufweisen, umfasst.

4. Verfahren nach Anspruch 1, wobei das Nutzen der bestimmten Werte zum Identifizieren eines oder mehrerer Proteine oder Proteininteraktionen, als Zielproteine oder Proteininteraktionen zum Targeting von Wirkstofftherapien, das Identifizieren von Proteininteraktionen, welche Verbindungen entsprechen, die mit bestimmten Werten größer als ein Grenzwert verknüpft sind, umfasst.

5. Verfahren nach Anspruch 1, wobei das Nutzen der bestimmten Werte zum Identifizieren eines oder mehrerer Proteine oder Proteininteraktionen, als Zielproteine oder Proteininteraktionen zum Targeting von Wirkstofftherapien, das Identifizieren von Proteinen, welche Knoten entsprechen, welche durch Verbindungen angeschlossen sind, welche mit bestimmten Werten größer als ein Grenzwert in verknüpft sind, umfasst.

6. Ein computerimplementiertes Verfahren zum Identifizieren von Zielproteinen zum Targeting von Wirkstofftherapien, umfassend:
Erfassen (S2-1) von Proteomdaten für einen Zielorganismus;
Speichern (S4-1) dieser Proteomdaten als Netzwerkdaten (40) innerhalb eines Computers (2), wobei die Netzwerkdaten (40) Daten umfassen, welche eine Vielzahl von Knoten und eine Vielzahl von Verbindungen zwischen den Knoten definieren, wobei die Knoten mit Proteinen verknüpft sind und die Verbindungen bezeichnend für Proteininteraktionen sind;
Verarbeiten (S4-3) der gespeicherten Netzwerkdaten (40), wobei der Computer (2) genutzt wird, um für jeden der Knoten des Netzwerks, welches durch die gespeicherten Netzwerkdaten (40) definiert ist, einen Wert zu bestimmen, welcher bezeichnend für die Anzahl von Wegen zwischen den Knoten in dem Netzwerk, welche über die Knoten verlaufen, relativ zu der Gesamtzahl der Wege zwischen den Knoten in dem Netzwerk ist; und
Nutzen der bestimmten Werte, die mit den Knoten, welche den Proteinen entsprechen, verknüpft sind, um ein oder mehrere Proteine als Zielproteine zum Targeting von Wirkstofftherapien zu identifizieren (S2-2).

7. Verfahren nach Anspruch 6, wobei das Verarbeiten (S4-3) für jeden der Knoten umfasst:
Identifizieren eines Knotensatzes, welcher direkt mit dem Knoten über eine Verbindung angeschlossen ist;
Identifizieren (S8-2) von Knotenpaaren des identifizierten Knotensatzes;
Bestimmen (S8-4 - S8-7) von Wegen über Knoten und Verbindungen, welche die identifizierten Knotenpaare anschließen, für jedes der identifizierten Knotenpaare; und
Berechnen (S8-10) des Verhältnisses von der Anzahl der bestimmten Wege, welche über einen Knoten verlaufen, relativ zu der Gesamtzahl der bestimmten Wege, als den Wert für den Knoten.

8. Verfahren nach Anspruch 7, wobei die Bestimmung der Wege zwischen Knotenpaaren die Bestimmung von Wegen zwischen Knotenpaaren, welche bis zu einer vorbestimmten Anzahl von Verbindungen besitzen, umfasst.

9. Verfahren nach Anspruch 8, wobei die Bestimmung von Wegen zwischen Knotenpaaren die Bestimmung von Wegen über Knoten umfasst, wobei die Wege jeden Knoten nicht mehr als einmal enthalten.

10. Ein Verfahren zur Herstellung eines therapeutischen Arzneimittels, umfassend:
Identifizieren eines oder mehrerer Zielproteine oder Zielproteininteraktionen, als Zielproteine oder Proteininteraktionen zum Targeting von Wirkstofftherapien durch die Durchführung eines Verfahrens (S2-2) nach einem der vorhergehenden Ansprüche;
Identifizieren (S2-6) eines oder mehrerer Präparate, welche mit dem einen oder mehreren identifizierten Zielproteinen oder Proteininteraktionen reagieren; und
Herstellen eines therapeutischen Arzneimittels, welches Präparate beinhaltet, welche als mit den identifizierten Zielproteinen oder Proteininteraktionen reagierend identifiziert sind.

11. Ein computerlesbares Medium, welches computerinterpretierbare Anweisungen speichert, welche, wenn sie durch einen programmierbaren Computer interpretiert werden, den Computer dazu veranlassen, ein Verfahren nach einem der Ansprüche 1 bis 9 auszuführen.

12. Eine Informationsverarbeitungsvorrichtung (2) zum Identifizieren von Zielproteinen oder Proteininteraktionen zum Targeting von Wirkstofftherapien, umfassend:
einen Datenspeicher (10), welcher konfiguriert ist, um Proteomdaten für einen Zielorganismus als Netzwerkdaten (40) zu speichern, welche eine Vielzahl an Knoten und eine Vielzahl an Verbindungen zwischen den Knoten definiert, wobei die Knoten mit Proteinen verknüpft sind und die Verbindungen bezeichnend für die Proteininteraktionen sind;
eine Verarbeitungseinheit (26), welche funktionsfähig ist, Netzwerkdaten (40) zu verarbeiten, welche in dem Datenspeicher (10) gespeichert sind, um für jede der Verbindungen des Netzwerkes, welches durch die gespeicherten Netzwerkdaten (40) definiert ist, einen Wert zu bestimmen, welcher bezeichnend für das Verhältnis der Anzahl der Wege zwischen den Knoten in dem Netzwerk, welche über die Verbindung verlaufen, relativ zu der Gesamtzahl der Wege zwischen den Knoten in dem Netzwerk ist; und
eine Ausgabeeinheit, welche funktionsfähig ist, Daten zum Identifizieren von einem oder mehreren Zielproteinen oder Proteininteraktionen zum Targeting von Wirkstofftherapien auf der Basis von Werten, welche mit Verbindungen verknüpft sind, welche durch die Verarbeitungseinheit (26) bestimmt sind, auszugeben.

13. Informationsverarbeitungsvorrichtung (2) nach Anspruch 12, wobei die Ausgabeeinheit durch das Identifizieren von Knoten, welche durch Verbindungen angeschlossen sind, welche mit bestimmten Werten größer als ein Grenzwert verknüpft sind, funktionsfähig ist, Daten auszugeben, welche ein oder mehrere Zielproteine zum Targeting von Wirkstofftherapien identifizieren.

14. Eine Informationsverarbeitungsvorrichtung (2) nach Anspruch 12, wobei die Ausgabeeinheit durch das Identifizieren von Verbindungen, welche mit bestimmten Werten größer als ein Grenzwert verknüpft sind, funktionsfähig ist, Daten auszugeben, welche ein oder mehrere Zielproteininteraktionen zum Targeting von Wirkstofftherapien identifizieren.

15. Informationsverarbeitungsvorrichtung (2) zum Identifizieren von Zielproteinen zum Targeting von Wirkstofftherapien, umfassend:
einen Datenspeicher (10), welcher konfiguriert ist, um Proteomdaten für einen Zielorganismus als Netzwerkdaten (40) zu speichern, welche eine Vielzahl an Knoten und eine Vielzahl an Verbindungen zwischen den Knoten definieren, wobei die Knoten mit Proteinen verknüpft sind und die Verbindungen bezeichnend für die Proteininteraktionen sind;
eine Verarbeitungseinheit (26), welche funktionsfähig ist, Netzwerkdaten (40) zu verarbeiten, welche in dem Datenspeicher (10) gespeichert sind, um für jeden der Knoten des Netzwerkes, welches durch die gespeicherten Netzwerkdaten (40) definiert ist, einen Wert zu bestimmen, welcher bezeichnend für das Verhältnis der Anzahl von Wegen zwischen den Knoten in dem Netzwerk, welche über die Knoten verlaufen, relativ zu der Gesamtzahl von Wegen zwischen den Knoten in dem Netzwerk ist; und
eine Ausgabeeinheit, welche funktionsfähig ist, Daten auszugeben, welche ein oder mehrere Zielproteine zum Targeting von Wirkstofftherapien auf der Basis von Werten identifizieren, welche mit Knoten verknüpft sind, welche durch die Verarbeitungseinheit (26) bestimmt sind.

## Revendications

1. Procédé mis en oeuvre par ordinateur pour l'identification de protéines cibles ou interactions de protéines cibles pour ciblage par des thérapies médicamenteuses comprenant les étapes consistant à :
obtenir (s2-1) des données de protéome pour un organisme à cibler ;
stocker (s4-1) lesdites données de protéome sous la forme de données de réseau (40) dans un ordinateur (2), lesdites données de réseau (40) comprenant des données définissant une pluralité de noeuds et une pluralité de liaisons entre lesdits noeuds, dans lequel lesdits noeuds sont associés à des protéines et lesdites liaisons sont indicatives d'interactions de protéines ;
traiter (s4-3) les données de réseau stockées (40) en utilisant l'ordinateur (2) pour déterminer, pour chacune des liaisons du réseau définie par les données de réseau stockées (40), une valeur indicative du rapport entre le nombre de chemins reliant les noeuds du réseau qui passent par cette liaison et le nombre total de chemins reliant lesdits noeuds au sein et dudit réseau ; et
utiliser lesdites valeurs déterminées associées audites liaisons correspondants à des interactions de protéines pour identifier (s2-2) une ou plusieurs protéines ou interactions de protéines comme protéines cibles ou interactions de protéines cibles pour ciblage par des thérapies médicamenteuses.

2. Procédé selon la revendication 1, dans lequel ledit traitement (s4-3) comprend, pour chacune desdites liaisons :
l'identification d'un premier et d'un deuxième noeud connectés par ladite liaison ;
l'identification d'un premier jeu de noeuds reliés directement audit premier noeud et d'un deuxième jeu de noeuds reliés directement audit deuxième noeud ;
l'identification (s11-2) de toutes les paires de noeuds comprenant des noeuds différents sélectionnés parmi lesdits premier et deuxième jeu de noeuds ;
la détermination (s11-4 - s11-9) pour chacune desdites paires de noeuds identifiés, des chemins passants par des noeuds et des liaisons reliant lesdites paires de noeuds identifiés ; et
le calcul (s11-10) en tant que ladite valeur pour une liaison, du rapport entre le nombre desdits chemins déterminés qui passent par ladite liaison et le nombre total desdits chemins déterminés.

3. Procédé selon la revendication 2, dans lequel ladite détermination des chemins reliant des paires de noeuds comprend la détermination de chemins entre des paires de noeuds présentant jusqu'à un nombre prédéterminé de liaisons.

4. Procédé selon la revendication 1, dans lequel l'utilisation desdites valeurs déterminées pour identifier une ou plusieurs protéines ou interactions de protéines comme protéines cibles ou interactions de protéines cibles pour ciblage par des thérapies médicamenteuses comprend l'identification d'interactions de protéines correspondant à des liaisons associées à des valeurs déterminées qui sont supérieures à une valeur seuil.

5. Procédé selon la revendication 1, dans lequel l'utilisation desdites valeurs déterminées pour identifier une ou plusieurs protéines ou interactions de protéines comme protéines cibles ou interaction cibles de protéines pour ciblage par des thérapies médicamenteuses comprend l'identification de protéines correspondants à des noeuds reliés par des liaisons associées à des valeurs déterminées qui sont supérieures à une valeur seuil.

6. Procédé mis en oeuvre par ordinateur pour l'identification de protéines cibles pour ciblage par des thérapies médicamenteuses comprenant les étapes consistant à :
obtenir (s2-1) des données de protéome pour un organisme à cibler;
stocker (s4-1) lesdites données de protéome sous la forme de données de réseau (40) dans un ordinateur (2), lesdites données de réseau (40) comprenant des données définissant une pluralité de noeuds et une pluralité de liaisons entre lesdits noeuds, dans lequel lesdits noeuds sont associés à des protéines et lesdites liaisons sont indicatives d'interactions de protéines ;
traiter (s4-3) les données de réseau stockées (40) en utilisant l'ordinateur (2) pour déterminer, pour chacun des noeuds du réseau défini par les données de réseau stockées (40), une valeur indicative du nombre de chemins entre les noeuds du réseau qui passe par ces noeuds par rapport au nombre total de chemins entre lesdits noeuds dans ledit réseau ; et
utiliser lesdites valeurs déterminées associées audit ne correspondant à des protéines pour identifier (s2-2) une ou plusieurs protéines en tant que protéines cibles pour ciblage par des thérapies médicamenteuses.

7. Procédé selon la revendication 6, dans lequel ledit traitement (s4-3) comprend, pour chacun desdits noeuds :
l'identification d'un jeu de noeuds reliés directement audit noeud au moyen d'une liaison ;
l'identification (s8-2) de paires de noeuds duditjeu de noeuds identifié;
la détermination (s8-4 - s8-7) pour chacune desdites paires de noeuds identifiées, de chemins passants par des noeuds ainsi que des liaisons reliant lesdites paires de noeuds identifiées; et
le calcul (s8-10) en tant que ladite valeur pour un noeud, du rapport entre le nombre desdits chemins déterminés passant par ledit noeud et le nombre total desdits chemins déterminés.

8. Procédé selon la revendication 7, dans lequel ladite détermination de chemins entre lesdites paires de noeuds comprend la détermination de chemins entre des paires de noeuds présentant jusqu'à un nombre prédéterminé de liaisons.

9. Procédé selon la revendication 8, dans lequel ladite détermination de chemins entre des paires de noeuds comprend la détermination de chemins via des noeuds dans laquelle lesdits chemins ne comprennent pas un noeud quelconque plus qu'une fois.

10. Un procédé de fabrication d'un médicament comprenant :
l'identification d'une ou plusieurs protéines cibles ou interactions de protéines cibles pour ciblage par des thérapies médicamenteuses pour ciblage par des thérapies médicamenteuses par la mise en oeuvre d'un procédé (s2-2) selon l'une quelconque des revendications précédentes ;
l'identification (s2-6) d'un ou de plusieurs composés qui réagissent avec la ou les protéines cibles ou interactions de protéines cibles identifiées ; et
la fabrication d'un médicament qui contient des composés identifiés comme réagissant avec les protéines cibles ou les interactions de protéines cibles identifiées.

11. Un support lisible par ordinateur stockant des instructions interprétables par ordinateur qui, lors de leur interprétation par un ordinateur programmable, provoquent la réalisation du procédé selon l'une quelconque des revendications 1 à 9 par l'ordinateur.

12. Un dispositif de traitement de l'information (2) pour l'identification de protéines cibles ou d'interactions de protéines cibles pour ciblage par des thérapies médicamenteuses, comprenant :
une mémoire de données (10) configurée pour stocker des données de protéome pour un organisme destiné à être ciblé sous forme de données de réseau (40) définissant une pluralité de noeuds et une pluralité de liaisons entre lesdits noeuds dans lequel lesdits noeuds sont associés à des protéines et lesdites liaisons sont indicatives d'interactions de protéines ;
une unité de traitement (26) susceptible de fonctionner pour traiter les données de réseau stockées (40) dans ladite mémoire de données (10) pour déterminer, pour chacune des liaisons du réseau définie par les données de réseau stockées (40), une valeur indicative du rapport entre le nombre de chemins entre les noeuds du réseau qui passent par la liaison et le nombre total de chemins entre lesdits noeuds dans ledit réseau ; et
une unité de sortie susceptible de fonctionner pour sortir des données identifiant une ou plusieurs protéines cibles ou interactions de protéines cibles pour ciblage par des thérapies médicamenteuses sur la base de valeurs associées à des liaisons déterminées par l'unité de traitement (26).

13. Dispositif de traitement de l'information (2) selon la revendication 12, dans lequel ladite unité de sortie est susceptible de fonctionner pour sortir des données identifiant une ou plusieurs protéines cibles pour ciblage par des thérapies médicamenteuses grâce à l'identification de noeuds reliés par des liaisons associées à des valeurs déterminées supérieures à une valeur de seuil.

14. Dispositif de traitement de l'information (2) selon la revendication 12, dans lequel ladite unité de sortie et susceptible de fonctionner pour sortir des données identifiant une ou plusieurs interactions de protéines cibles pour ciblage par des thérapies médicamenteuses grâce à l'identification de liaisons associées avec des valeurs déterminées supérieures à une valeur de seuil.

15. Dispositif de traitement de l'information (2) pour l'identification de protéines cibles pour ciblage par des thérapies médicamenteuses, comprenant :
une mémoire de données (10) configurée pour stocker des données de protéome pour un organisme destiné à être ciblé sous forme de données de réseau (40) définissant une pluralité de noeuds et une pluralité de liaisons entre lesdits noeuds dans lequel lesdits noeuds sont associés à des protéines et lesdites liaisons sont indicatives d'interactions de protéines ;
une unité de traitement (26) susceptible de fonctionner pour traiter les données de réseau stockées (40) dans ladite mémoire de données (10) pour déterminer, pour chacun parmi les noeuds du réseau définis par les données de réseau stockées (40), une valeur indicative du rapport entre le nombre de chemins entre les noeuds du réseau qui passent par lesdits noeuds et le nombre total de chemins entre lesdits noeuds dans ledit réseau ; et
une unité de sortie susceptible de fonctionner pour sortir des données identifiant une ou plusieurs protéines cibles pour ciblage par des thérapies médicamenteuses sur la base des valeurs associées aux noeuds déterminés par l'unité de traitement (26).
